# EUROPEAN PATENT APPLICATION

(11) **EP 4 488 682 A1**
(43) Date of publication of application: **08.01.2025**
(21) Application number: 23183879.8
(22) Date of filing: 06.07.2023
(51) Int. Cl.: G01N 33/543, G01N 33/557

(54) **METHOD AND SYSTEM FOR ANALYSING A BIOLOGICAL SAMPLE**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

A method is provided for analysing a biological sample (200, 308) with at least one target analyte (208). The method comprises the steps: adding to the biological sample (200, 308) at least a first marker (202) and a second marker (204), wherein the first marker (202) is configured to bind specifically to a first part of the at least one target analyte (208) and the second marker (204) is configured to bind specifically to a second part of the at least one target analyte (208); and determining the presence of the target analyte (208) in the biological sample (202, 308) based on detecting the first marker (202) and the second marker (204) in the biological sample (200, 308). In a further aspect a system (300) is provided configured to perform the method.

## Description

### Technical field

The invention relates to a method and system for analysing a biological sample with a target analyte with a first marker and a second marker.

### Background

Diverse methods for marking and imaging biological samples are known in the art. Often, these methods rely on markers comprising affinity reagents such as antibodies that are labelled in order to identify target analytes in the biological samples. This approach inherently relies on affinity reagents having a high affinity only to the respective target analyte. However, frequently this is not the case with affinity reagents and therefore markers exist that show an at least moderate degree of cross-reactivity towards a plurality of different so called OFF-targets. The fact that the majority of antibodies displays cross-reactivity is therefore a notable challenge in life science research generating false-positive results, increasing costs, and uncertainties in data interpretation.

Especially in the context of high-throughput and high-plex assays, where a large number of samples is analysed with a large number of markers, any cross-reactivity can have large consequences for reliability and reproducibility of these assays. As an example, antibodies, particularly hybridoma generated monoclonal antibodies, can regularly have cross-reactivity with several target analytes. In recent years the availability of commercial antibodies has quickly increased. Antibodypedia.com lists more than 11,000 antibodies from 65 vendors against epidermal growth factor receptor (EGFR). While efforts have been undertaken to improve antibody validation protocols, it is clear that it is very difficult for the scientific community to keep pace with the rapid rise in commercial antibody availability.

Multi-plex and high-plex proteomics both in the context of liquid biopsies that may be derived from blood samples, saliva, lymph, or liquor, as well as in the context of spatial proteomics in tissue sections and 3-dimensional samples like organoids is becoming increasingly important. The human blood proteome for example holds information about physiological and pathophysiological processed in the donor's body. High-plex plasma proteomics is therefore of considerable interest for the stratification of patient populations, early disease detection, and prevention. The analysis of blood proteins is, however, complicated by the fact that high abundance plasma proteins like serum albumin and globulins are significantly more frequent than low abundance proteins like for example thyroid-stimulating hormone, which may be of particular interest. The dynamic range may be as high as 10¹².

Similarly, in the context of spatial biology samples, which are typically tissue sections the highest interest may be related to finding rare cells that are particularly important.

For both cases, liquid and solid biological sample, non-imaging-based methods exist to profile said samples. Yet, imaging-based readouts are generally time-consuming.

### Summary

It is an object to provide a method and a system for reliably and efficiently analysing a biological sample with a target analyte.

The aforementioned object is achieved by the subject-matter of the independent claims. Advantageous embodiments are defined in the dependent claims and the following description.

A method is provided for analysing a biological sample with at least one target analyte. The method comprises the steps: a) adding to the biological sample at least a first marker and a second marker, wherein the first marker is configured to bind specifically to a first part of the at least one target analyte and the second marker is configured to bind specifically to a second part of the at least one target analyte; and b) determining the presence of the target analyte in the biological sample based on detecting the first marker and the second marker in the biological sample.

By using at least two markers, each marker being specific to a different part of the target analyte, the method enables determining the presence of the target analyte in the biological sample with high specificity. In particular, this enables drastically reducing the risk of misidentifying of the target analyte due to cross-reactivity of the markers towards other target analytes.

The biological sample may be a tissue section, for example. Alternatively, the biological sample may be a liquid sample such as a liquid biopsy, in particular solid components thereof. The target analyte may be a proteomic component, i.e. a protein of the biological sample, or a genomic component, i.e. a nucleic acid (DNA, RNA) sequence in particular a sequence that folds into a structure. The first part and the second part of the target analyte may also be termed as different epitopes of the target analyte. Determining the presence of the target analyte in the biological sample based on detecting the first marker and the second marker in the biological sample, e.g. with a microscope, may preferably include determining the location of the target analyte in the biological sample.

In the sense of this document "marker" is preferably used to denote both a single molecule used as marker and a collection of identical molecules used as marker. A "marker" in the sense of this document is the combination of an affinity reagent configured to attach to a predetermined structure also referred to as a target molecule or an analyte and/or a "reporter", which may also be referred to as a "detectable label".

In the sense of the present document, an analyte may be at least one of a protein, peptide, neurotransmitter, hormone, small molecule, nanostructure, and a secondary structure of a nucleic acid. More specifically, a nucleic acid sequence stretch that may bound by an oligonucleotide sequence via hybridization is not an analyte in the sense of this document. Likewise, an affinity reagent in the sense of this document is at least one of the following an antibody, an antibody fragment, a nanobody, an aptamer, wherein aptamer may also refer to modified aptamers like SOMAmers and aptabodies, which are modified either by changes to the backbone or modification of the nucleobases. In the sense of the present document, an aptamer may be used as an affinity reagent for a marker used in conjunction with the method in order to mark a nucleic acid stretch that forms a secondary structure, so as long as the interaction of the affinity reagent and the target is not mediated via hybridization of complementary nucleic acid sequences but by paratope-epitope type interaction. Thus, the marker may preferably comprise an affinity reagent that is a nucleic acid aptamer having a complex secondary and/or tertiary structure and the nucleic acid aptamer is configured to specifically bind to the target analyte due to its complex secondary and/or tertiary structure rather than complementary base pairing.

"Aptamer": In the sense of this document "aptamer" preferably refers to a small affinity reagent that is typically based on nucleic acids - either naturally occurring RNA, DNA or artificial ones XNAs, but may in some cases also be based on a peptide backbone. In the sense of this document "aptamer" also refers to derivatives of aptamers such as SOMAmers or aptabodies, which are modified with modifications typically found on proteins such as glycosylation for example.

"Sample": In the sense of this document "sample" preferably refers to a biological sample which may also be named a biological specimen including, for example blood, serum, plasma, tissue, bodily fluids (e.g. lymph, saliva, semen, interstitial fluid, cerebrospinal fluid), feces, solid biopsy, liquid biopsy, explants, model organism embryos (e.g. zebrafish, Drosophila), entire model organisms (e.g. zebrafish larvae, Drosophila embryos, C. elegans), cells (e.g. prokaryotes, eukaryotes, archea), multicellular model organisms (e.g. Volvox), suspension cell cultures, monolayer cell cultures, 3D cell cultures (e.g. spheroids, tumoroids, organoids derived from various organs such as intestine, brain, heart, liver, etc.), a lysate of any of the aforementioned, a virus. In the sense of this document "sample" further refers to a volume surrounding a biological sample. Like for example in assays, where secreted proteins like growth factors, extracellular matrix constituents are being studied the extracellular environment surrounding a cell up to a certain assay-dependent distance, is also referred to as the "sample". Specifically, affinity reagents brought into this surrounding volume are referred to in the sense of this document as being "introduced into the sample".

In the step of adding the markers to the biological sample (step a)), the markers may, in particular, be introduced into the biological sample. This may include permeabilising the biological sample, for example.

Preferably, the markers are detected optically based on light emitted by the markers. Thus, the markers may comprise optically detectable labels, which may comprise fluorochromes. This enables easy detection of the markers. When one of the markers is bound to the target analyte, light emitted by the marker may be detected, attributed to the target analyte and the presence of the target analyte determined.

Preferably, prior to the step of adding the markers, the biological sample is fixed in place. Particularly, components of the biological sample, including the target analyte, are fixed in their location relative to each other. This enables efficiently determining the location of the target analyte in the biological sample. In particular, when the biological sample is a liquid sample, fixing in place may include crosslinking solid components of the biological sample with each other, embedding the solid components in a hydrogel, or linking the solid components to a surface. Such a surface may be a surface of a microfluidic device, through which the liquid sample is streamed, in order to fix or deposit the sample in place.

In a preferred embodiment, the first marker and the second marker are added to the biological sample simultaneously or subsequently. This enables flexibly carrying out the method. When the markers are added subsequently, they are added at separate or different times to the biological sample. When the markers are added simultaneously, they are added at the same time.

Preferably, the first marker is removed prior to adding the second marker, in particular, in case the markers are added to the biological sample subsequently. This enables efficient cyclical staining of the biological sample with a plurality of markers in order to determine the presence of the target analyte. When removing one of the markers, the entire marker may be removed, or only an optically detectable label of the marker may be removed. The removal of the marker results in the target analyte being no longer associated with at least the optically detectable label and therefore it is no longer possible to detect the presence of the target analyte based on the removed marker. Markers may be oligonucleotide- or peptide-based and removed by adding a degradation agent such as a nuclease or a protease. Preferably, the degradation agent is specific to the particular marker. Preferably, marker removal is performed by degrading the marker with a degradation agent. The degradation agent could comprise at least one of the following: a DNase, RNase, endo-/exonuclease. Thus, preferably, marker removal is performed by degrading the marker with a degradation agent, the degradation agent may comprise at least one of the following: a DNase, RNase, endo-/exonuclease

Preferably, at least one optical readout of the biological sample with the first marker and/or the second markers is generated. This enables determining the location of the target analyte within the image and within the biological sample. In particular, the optical readout is generated prior to determining the presence of the target analyte. The presence of the target analyte is then based on the generated optical readout. The optical readout is preferably generated over time. The optical readout may comprise several discrete images or a continuous image stream over time, for example.

The method may preferably be carried out in a cyclic fashion, i.e. over multiple cycles of staining, imaging, blanking, wherein blanking may be performed by inactivating the labels e.g. through label removal, bleaching, or quenching or by removing the markers (marker removal). In the case of cyclic operation at least one optical readout at a single time point may be collected per cycle or alternatively an optical readout time series may be collected per cycle. The former allows multi- or high-plex proteomics to be performed using single colour labels or combinatorial encoding. The latter allows the same but in addition also enables (A) locating the marker with higher spatial precision and (B) enables the derivation of kinetic information since the binding of markers to targets is monitored over time. In this way for each target multiple binding events may be monitored and kinetic information may be derived. As stated before each target may be bound by at least two and up to n affinity reagents, which increases the specificity of the assay.

Preferably, the presence of the target analyte in the biological sample is determined based on detecting the first marker and the second marker over time, for example, in an optical readout. For example, light emitted by the markers is detected over time when the target analyte is bound to the respective marker. This might in particular be accomplished in a cyclic fashion by obtaining a readout trace over multiple rounds of staining, imaging, and marker removal. This enables determining the presence of the target analyte with increased specificity and certainty.

It is particularly preferred that the presence of the target analyte in the biological sample is determined based on a frequency and/or a duration of light emitted by the first marker and the second marker over time. This enables determining the presence of the target analyte with increased specificity and certainty. In particular, the presence of the target analyte in the biological sample is determined in case the frequency and/or duration of light emitted by the markers is over a predetermined threshold. For example, markers that bind to the target analyte only transiently or with only a low affinity may also only bind for short amounts of time to the target analyte.

Preferably, based on the frequency and/or the duration of light emitted by the first marker and the second marker equilibrium constants, particularly binding constants, of the association (ON-rate, *k*_{ON}) and dissociation (OFF-rate, *k*_{OFF}) of the first marker and second marker with the target analyte are determined, and the presence of the target analyte in the biological sample is determined based on the equilibrium constants. In particular, the determination of the equilibrium constants may be based on the frequency and/or duration of light emitted by the markers over time. Thus, by determining kinetics of the binding of the markers to the target analyte, weak off-target binding or non-specific interactions between markers and off-targets may be recognized as background binding or cross-reactivity and differentiated from binding to the actual target analyte. For example, the presence of the target analyte may be determined in case a binding constant between a particular one of the markers and the target analyte is above a particular threshold.

Preferably, environmental conditions that the biological sample is in are set based at least on one physicochemical parameter of the first marker and/or the second marker. The physicochemical parameters may include pKa or pl of the respective marker. The environmental conditions may include a type or a concentration of a buffer the biological sample is in, or the temperature of the biological sample. In particular, the environmental conditions may be set such that the first marker and/or the second marker bind to the target analyte with high affinity. This enables determining the presence of the target analyte with high specificity. The environmental conditions may further or alternatively include a concentration of a competitor, such as a polyanionic competitor, in order to prevent weak binding or non-specific binding of markers to components of the biological sample.

Preferably, the environmental conditions are set such that the first marker and/or the second marker are only transiently binding to the target analyte. This enables determining the presence of the target analyte with high specificity and efficiency, especially in case the first marker and the second marker are added to the biological sample simultaneously.

The method disclosed in the present document may therefore take advantage of affinity reagents that display only moderate affinity to their targets and only moderate specificity. As target analytes are recognized by binding n affinity reagents to 1 target analyte, i.e. n:1 detection the method offers an exponential increase in specificity when a higher number of affinity reagents are used to detect the target analyte. This is true even, when each affinity reagent does display cross-reactivity so long as the n affinity reagents used to detect the said 1 target analyte do not display exactly the same cross-reactivity. While most affinity reagents, like antibodies, nanobodies, and aptamers are cross-reactive, their cross-reactivity can usually be expected to be substantially different, i.e. towards substantially different sets of OFF-targets.

Preferably, the first marker and the second marker each comprise an affinity reagent configured to specifically bind to the respective part of the target analyte and the affinity reagent is an aptamer or an antibody fragment, such as a single-domain antibody or a nanobody. This enables flexible adaptation of the method to different target analytes. Preferably, the first marker is one of the aptamer or the antibody fragment and the second marker is the other of the aptamer or the antibody fragment. This enables determining the presence of target analyte with particularly high specificity.

Preferably, the first marker and the second marker each comprise an optically detectable label, for example a fluorophore, and the optically detectable labels of the first marker and the second marker are optically distinguishable from each other. Amongst others, this enables efficiently determining the association and dissociation of markers with the target analyte. In particular, the labels of the first marker and the second marker may differ in their optical properties such as emission wavelength, excitation wavelength and/or fluorescence lifetime.

The optically detectable label may for example comprise an oligonucleotide or DNA nanostructure backbone and may be a single colour label comprising a single dye or a combination of dyes (combinatorial label). The optically detectable label may be conjugated directly or indirectly via hybridization to a barcode sequence to the affinity reagent. The backbone of the label and/or the aptamer may also be an artificial nucleic acid (XNA).

Preferably, at least a further marker is added to the biological sample. In particular, the further marker may be configured to bind specifically to the target analyte, preferably, a further part of the target analyte. This enables increasing the specificity of determining the presence of the target analyte. The further marker may be added subsequently to the first and second markers. The use of aptamers is particularly preferred since they may be directly generated with high specificity to particular target analytes, for example by a SELEX method. In an alternative, the further marker is configured to specifically bind to a further target analyte in order to determine the presence of the further target analyte in the biological sample. Thus, the biological sample may be stained iteratively with at least three different markers in order to determine the presence of the target analyte. Additional markers may be used to determine the presence of further target analytes.

The use of aptamers is particularly preferred since their backbone is usually a nucleic acid and therefore displays differential sensitivity to nucleases (e.g. DNase I, RNase I, endo-/exonucleases) as compared to the protein analytes. This allows particularly gentle cyclic labelling with marker removal by substantially degrading aptamers, which has been described before, particularly a suitable method for analysing a biological sample, in the European patent application with the application number 23178065.1, the complete content thereof being included here by reference. The use of aptamers is particularly preferred since they can be selected, in particularly designed, against an epitope of choice in contrast to antibodies, in which case the immune system of the animal producing the antibody randomly selects the epitope. This is important, when affinity reagents shall be generated against specific posttranslational modifications such as phosphorylation, ubiquitination, acetylation, methylation, glycosylation, prenylation, sulfation, disulfide bonds, proteolytic cleavage, sumoylation shall be generated.

Preferably, the optical readout is generated by means of a microscope, in particular a light microscope, a fluorescence microscope, a confocal scanning microscope. This enables generating an image of the biological sample and determining the location of the target analyte in the biological sample efficiently.

In a further aspect a system is provided configured to perform the method set out above. The system may comprise the microscope for generating the optical readout. In addition, the system may comprise a microfluidic device, in particular, for analysing liquid samples. The microfluidic device may comprise a surface for fixing the solid components of the liquid sample. The surface may be observable with the microscope in order to generate the optical readout of the sample fixed on the surface.

### Short Description of the Figures

Hereinafter, specific embodiments are described referring to the drawings, wherein:
- Figure 1: is a flow chart of a method for analysing a biological sample with at least one target analyte,
- Figure 2: is a schematic view of the biological sample with markers over time, and
- Figure 3: is a schematic view of a system configured to perform the method according to the flow chart of Fig. 1,
- Figure 4: is a schematic showing a cyclic process with one time point acquisition or time series (continuous) acquisition per cycle, and
- Figure 5: is a schematic illustrating different modes in which the process according to Fig. 4 may be performed.

### Detailed Description

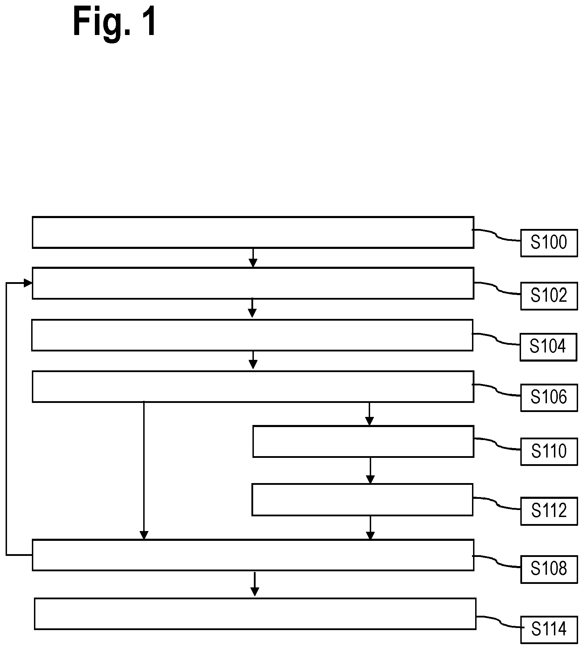

Figure 1 is a flow chart of a method for analysing a biological sample with at least one target analyte. The biological sample may be a tissue section, for example, or a liquid sample, such as a liquid biopsy. The target analyte may be a particular protein or a nucleic acid, including mRNA or genomic DNA, of the biological sample.

The method starts in a step S100. In step S102, the biological sample with a target analyte is provided. In case the biological sample is a tissue section, the biological sample may be provided on a sample holder such as a glass slide or a microscope slide, a well plate, or a microfluidic chip. In addition, the tissue section may be expanded. In particular in case the biological sample is a liquid sample, the solid components of the liquid sample, such as proteins and nucleic acids, and including the target analyte, may be fixed, for example, to a surface. The surface may be a glass slide functionalised in order to enable attachment of the solid components. Additionally or alternatively, the solid components may be cross-linked with each other, or embedded in a hydrogel. The fixing of the components of the biological sample results in them fixed in place relative to each other. The fixing of the solid components may be done in a microfluidic device through which the liquid sample is streamed and which provides an attachment surface or a fixing space in which the fixed sample may be visually observed. The biological sample may also be expanded at this stage or at a later stage during the method.

In step S104, at least a first marker and a second marker are added to the biological sample. The first marker and the second marker are specific to the target analyte of the biological sample. Specifically, the first marker and the second marker each comprise an affinity reagent, such as an aptamer or an antibody fragment, that specifically binds to a part of the target analyte. Thus, the markers comprise affinity reagents with a high affinity to the respective part of the target analyte. Further, the first marker binds specifically to a first part of the target analyte. In contrast, the second marker binds specifically to a second part of the target analyte; the first part being different to the second part.

For example, in case the target analyte is a protein, the first part may be a first epitope that an antibody fragment of the first marker specifically binds to. In this case, the second part may be a second epitope that an antibody fragment of the second marker specifically binds to.

Thus, adding the first and second marker in step S104 results in binding of the markers to the target analyte. The biological sample may clearly comprise a large number of copies of the target analyte and similarly, a large number of copies of the first and second marker may be added to the biological sample.

The first and the second marker further each comprise an optically detectable label such as a fluorophore. Preferably, the optically detectable label of the first marker differs in its optical properties from the optically detectable label of the second marker. Thus, the first marker and the second marker are optically distinguishable. The optical properties may include an excitation wavelength, an emission wavelength, or a fluorescence lifetime.

Optionally, the step S104 may comprise introducing the markers into the biological sample. For example, in case the biological sample is a tissue section, the biological sample may be permeabilised in order to facilitate the introduction of markers into cells of the biological sample.

In step S106, an optical readout, in particular an image, is generated of the biological sample with the target analytes and markers. Upon binding of the markers to the target analyte, the optically detectable label of the markers is associated with the target analyte and the target analyte may be detected visually as a point source of light. The marker may not be detected or only detected as diffuse nonpoint background noise, when the marker is not bound to the target analyte.

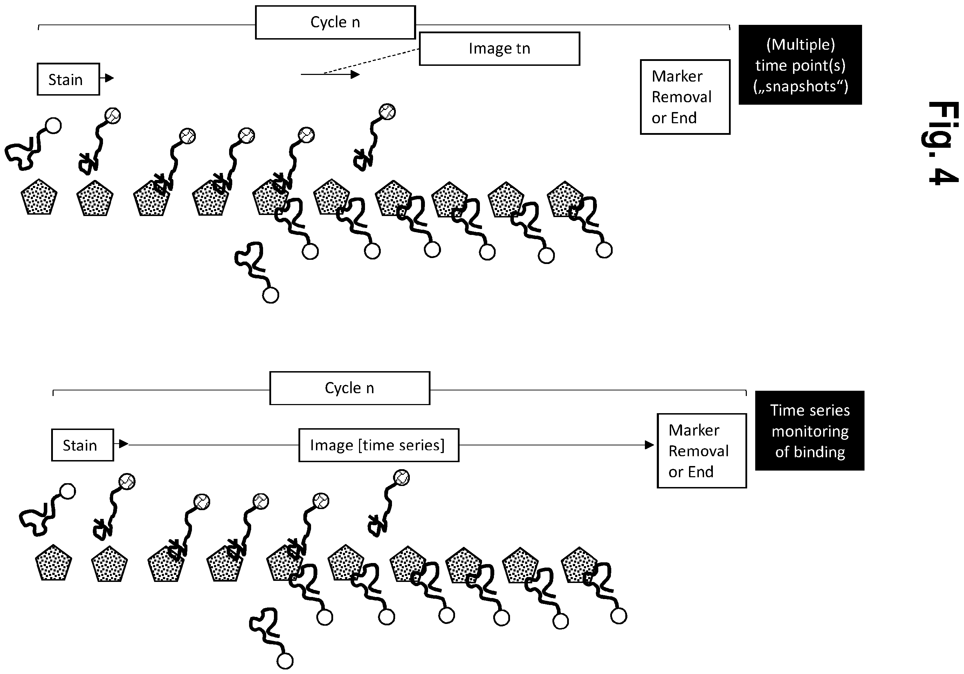

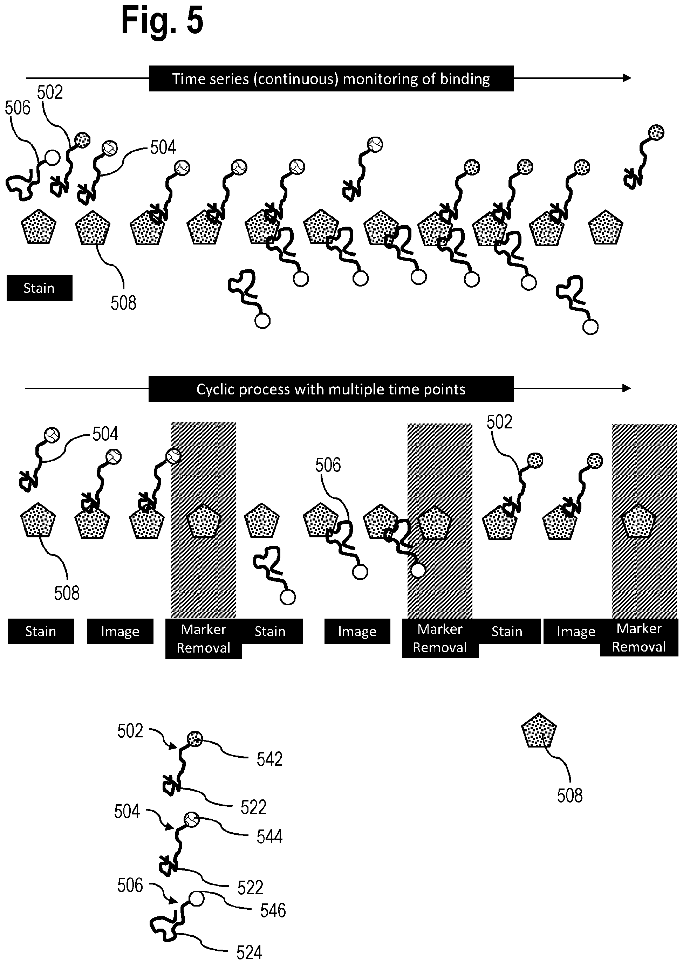

Preferably, the optical readout is generated over time. Thus, the optical readout may be a sequence of images of the biological sample generated at predetermined time intervals. Alternatively, the optical readout may be a continuous image or an image stream. The optical readout may be generated by means of a microscope configured to illuminate the biological sample at least with excitation light. This is further detailed in Figures 4 and 5.

In step S108 the presence of the target analyte in the biological sample is determined based on the detecting the first and second marker in the biological sample. Specifically, the presence of the target analyte may be determined based light emitted by the first and second markers associated with the target analyte.

In case the optical readout is an image of the biological sample, the detection of the markers associated with the target analyte may enable determining the location of the target analyte based on the location of the light emitted by the markers associated with the target analyte within the biological sample.

The use of the first marker in combination of the second marker results in a higher confidence of correctly determining the presence of the target analyte. For example, in case both markers have a cross-reactivity to other components of the biological sample, the presence of the target analyte is only determined if both markers are associated with the target analyte.

In case the optical readout is generate over time, the duration and/or frequency that the first marker and the second marker are associated with the target analyte may be determined. For example, in case the markers have a cross-reactivity to other components of the biological sample or in case the markers only have average affinity to the target analyte, the markers may only transiently bind to the target analyte. In this case, the presence of the target analyte may still be reliably determined by determining the duration and/or frequency that the first and second markers are both associated with each other at the target analyte. Thus, the location within the biological sample the first and second markers are co-detected at with increased duration and/or frequency may be determined as the location of the target analyte. A predetermined threshold for duration and/or frequency may be set in order to determine the location of the target analyte within the biological sample.

Further, based on the duration and/or frequency of light emitted from the first and second markers equilibrium constants, in particular binding constants, may be determined for the association and dissociation of the first marker and the second marker with the target analyte. Based on these determined constants the presence or location of the target analyte may be determined. For example, in case both markers have a high binding coefficient to a compound at a location within the biological sample, that compound may be determined to be the target analyte. A predetermined threshold for the binding coefficients may be set in order to determine the location of the target analyte within the biological sample.

In an alternative example of the method, only the first marker is added to the biological sample in step S104 following the step S102. The optical readout of the biological sample is then generated in the step S106 with only the first marker. In a step S110 the second marker is added similarly to what is described for step S104 and a second optical readout is generated in a step S112 similarly to what is described for step S106. In step S108 both optical readouts may then be used to determine the presence of the target analyte based on both, the first marker and the second marker similarly as to what is described for step S108 above. By separating the addition of markers in steps S104 and S110, the number of individual markers is reduced that are present in the optical readouts at the same time. This is advantageous in case a plurality of different target analytes is marked with markers at the same time in steps S104 and S110 or in case the target analyte is present in the biological sample in a very large number of copies.

The number of markers present in the biological sample may also be reduced by removing the first marker prior to step S110. This may be achieved by adding a degradation agent such as a protein that digests the marker, or parts thereof. Removing the marker results at least in the label of the marker being no longer associated with the respective target analyte.

Optionally, the environmental conditions the biological sample is in may be adjusted or set based on a physicochemical parameter of the first and/or second marker. The affinity of affinity reagents may be influenced by the environmental conditions they are in. For example, the affinity of antibodies may be influenced by buffer type or concentration, and temperature. In another example, the pl of the affinity reagent of the first marker may be taken into account when setting the pH the biological sample is in. This ensures that the biological sample is in conditions in which the markers bind at least transiently to the respective target analyte. Setting the environmental conditions may be part of steps S102, S104 and/or S110.

The method may be repeated iteratively after step S108 beginning again in step S102 with at least a further marker specific to a further target analyte. Prior to adding the further marker, the first and second markers are preferably removed.

Alternatively, the further marker may be added together with the first and second marker. In this case, the further marker may be specific to a further part of the target analyte, or to the further analyte.

In a further alternative, the further marker may be added after adding the second marker and generating the optical readout in steps S110 and S112. Prior to step S108 an optical readout is generated with the further marker. In this case, the further marker is preferably specific to a further part of the target analyte.

The method ends in step S114.

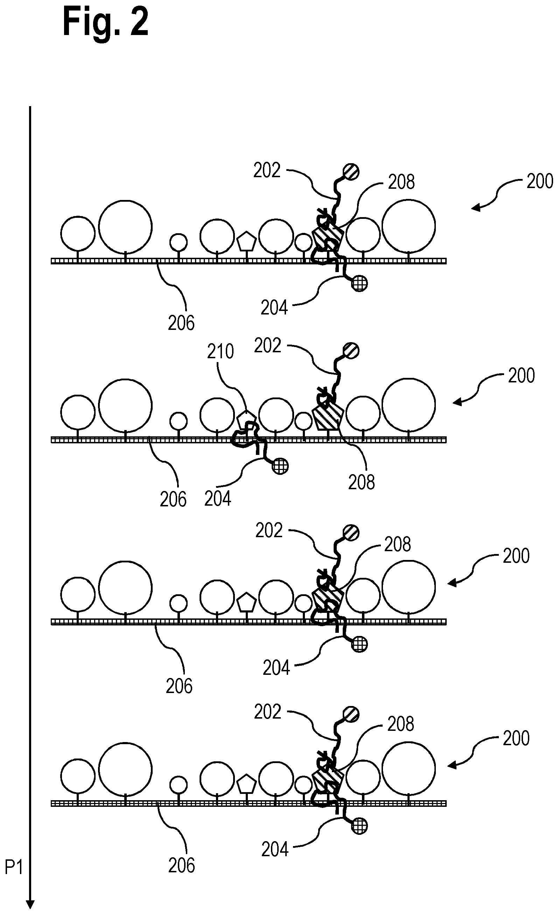

Figure 2 is a schematic view of a biological sample 200 with markers 202, 204 over time. The biological sample 200, in particular its solid components, is fixed on a surface 206. The arrow P1 is a timeline. Thus, Figure 2 shows a series of optical readouts of the biological sample 200 over time. A first marker 202 and a second marker 204 are configured to attach to a respective first part and a second part of a target analyte 208 of the biological sample 200 with high affinity. In three of the four views of the time series, the markers 202, 204 are attached to the target analyte 208. In the second view from the top in Figure 2, the second marker 204 is attached to a further component 210 of the biological sample. The second marker 204 may exhibit a low affinity to the component 210 and therefore transiently bind to it. Nevertheless, by observing the entire time series, the presence and location of the target analyte 208 may be determined based on the duration the first and second markers 202, 204 were located at the location of the target analyte 208 within the biological sample 200. Even if only the second from the top view were observed, the divergence of the location at which the first and the second markers 202, 204 are observed would indicate that the presence and location of the target analyte 208 was not and cannot be unambiguously determined from that view alone. The term location of markers 202, 204 in this context in particular, might be understood as a maximal distance between the location of the first marker 202 and the location of the second marker 204 in the biological sample 200. This distance might depend on the characteristics and the maximal spatial extend of the target analyte 208 and might be set to a predetermined threshold of e.g. 500 nm. Thus, by using two markers 202, 204, the presence and location of the target analyte 208 may be robustly determined. Depending on the mode in which the method is used, the labelling of the analytes may be substantially stable during the observation time or the labelling may be dynamic, i.e. markers may associate and dissociate repeatedly while the sample is being monitored. In the latter case this effectively leads to "blinking" of the target, which allows localization to be performed with super-resolution up to the point where individual molecules can be imaged and the binding of markers can be assigned to an individual target molecule.

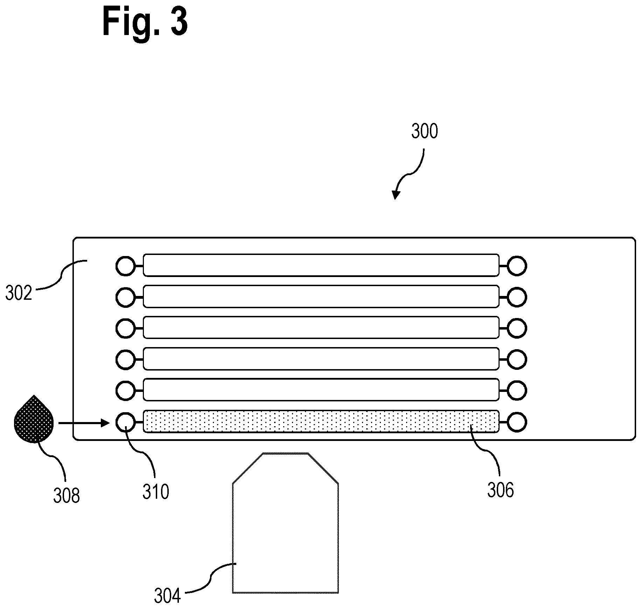

Figure 3 is a schematic view of a system 300 configured to perform the method for analysing a biological sample with a target analyte. The system 300 comprises a microfluidic device 302 and a microscope 304. The microfluidic device comprises a plurality of flow channels 306 for containing a biological sample with a target analyte. For example, a liquid sample 308 may be streamed into the flow channel 306 via an inlet port 310. The liquid sample 308 may then be fixed to a surface of the flow channel 306, for example. Markers or other reagents may equally be streamed into the flow channel 306 via the inlet 310 in order to add them to the fixed sample 308. The fixed sample 308 may then be observed and an optical readout be generated in the flow channel 306 by means of the microscope 304.

Figure 4 schematically depicts different ways in which the method can be executed. The method may be used for example with a cyclic staining, imaging, blanking process, wherein blanking may be achieved by, for example, marker removal. During the "image" step of the process (optical readout) a discrete image or single time points may be recorded resulting in a time series, in which time point n corresponds to cycle n, and time point n+1 corresponds to cycle n+1 (exemplarily shown on the left side of Fig. 4).

Alternatively, each "image" step may contain a time series consisting itself of multiple time points (exemplarily shown on the right side of Fig. 5). In this case time series n with time points e-h (e to h being time point identifiers) corresponds to cycle n, and time series n+1 with time points m-p (m to p being time point identifiers) corresponds to cycle n+1. The latter mode of operation allows a set of markers binding to one target analyte to be introduced in the "stain" step and then the association and dissociation of said marker from said set of markers to be monitored over time, allowing not only combinatorial encoding but also the derivation of kinetic information such as ON-/OFF-rates and equilibrium constants.

This is further schematically illustrated in Figure 5. In the example of Figure 5, a target analyte 508 (pentagon with speckle pattern) is assigned to a set of markers comprising three markers 502, 504, 506. The first marker 502 comprising a first aptamer 522 (affinity reagent) against a first epitope, and a first label 542. The second marker 504 comprising the first aptamer 522 against the first epitope, and a second label 544, i.e. same specificity but different label. The third marker 506 comprising a second aptamer 524 against a second epitope and a third label 546. This effectively allows to (A) encode the identity of the analyte 508 in a combinatorial code, which may be a combination or a permutation, and (B) to probe the identity of the analyte 508 with two distinct aptamers 522, 524 raising the specificity of the assay. Using this configuration of the assay the method may now be carried out such that all markers 502, 504, 506 are introduced in the "stain" step and then a time series is recorded to continuously monitor the binding and dissociation of the markers to the target. This is advantageous, if kinetic information about the binding of the markers 502, 504, 506 is sought after to differentiate ON-target vs. OFF-target binding or when the kinetic information as such is used as a further vector to encode information about the identity of the interaction. This case is shown time series monitoring of binding on the top of Figure 5.

In the bottom of Figure 5 a cyclic method is shown, wherein each marker 502, 504, 506 from said set of markers is introduced separate from each other in three separate rounds (each round may still contain many markers albeit from different sets i.e. against different target analytes). In this case the first marker 502 is introduced in the first, the second marker 504 in the second, and the third marker 506 in the third round. The readout is performed in each round during the "image" step or optical readout. Markers are removed before the next round.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 200: Biological sample
- 202, 502: First marker
- 204, 504: Second marker
- 506: Third marker
- 206: Surface
- 208, 508: Target analyte
- 210: Compound of the biological sample
- 300: System for analysing the biological sample
- 302: Microfluidic device
- 304: Microscope
- 306: Flow channel
- 308: Liquid sample
- 310: Liquid inlet
- 522, 524: Affinity reagents, aptamer
- 542, 544, 546: Label, fluorochrome

## Claims

1. A method for analysing a biological sample (200, 308) with at least one target analyte (208), comprising the steps:
a) adding to the biological sample (200, 308) at least a first marker (202) and a second marker (204), wherein the first marker (202) is configured to bind specifically to a first part of the at least one target analyte (208) and the second marker (204) is configured to bind specifically to a second part of the at least one target analyte (208), and
b) determining the presence of the target analyte (208) in the biological sample (200, 308) based on detecting the first marker (202) and the second marker (204) in the biological sample (200, 308).

2. The method according to claim 1, wherein prior to step a) the biological sample (200, 308) is fixed in place.

3. The method according to one of the preceding claims, wherein the first marker (202) and the second marker (204) are added to the biological sample (200, 308) simultaneously or subsequently.

4. The method according to one of the preceding claims, wherein first marker (202) is removed prior to adding the second marker (204).

5. The method according to one of the preceding claims, wherein at least one optical readout of the biological sample (200, 308) with the first marker (202) and/or the second marker (204) is generated.

6. The method according to one of the preceding claims, wherein the presence of the target analyte (208) in the biological sample (200, 308) is determined based on detecting the first marker (202) and the second marker (204) over time.

7. The method according to any one of the preceding claims, wherein the presence of the target analyte (208) in the biological sample (200, 308) is determined based on a frequency and/or a duration of light emitted by the first marker (202) and the second marker (204) over time.

8. The method according to claim 7, wherein based on the frequency and/or the duration of light emitted by the first marker (202) and the second marker (204) equilibrium constants of the association and dissociation of the first marker (202) and second marker (204) with the target analyte (208) are determined, and the presence of the target analyte (208) in the biological sample (200, 308) is determined based on the equilibrium constants.

9. The method according to one of the preceding claims, wherein environmental conditions that the biological sample (200, 308) is in are set based at least on one physicochemical parameter of the first marker (202) and/or the second marker (204).

10. The method according to claim 9, wherein the environmental conditions are set such that the first marker (202) and/or the second marker (204) are only transiently binding to the target analyte (208).

11. The method according to one of the preceding claims, wherein the first marker (202) and the second marker (204) each comprise an affinity reagent configured to specifically bind to the respective part of the target analyte (208) and the affinity reagent is an aptamer or an antibody fragment.

12. The method according to one of the preceding claims, wherein the first marker (202) and the second marker (204) each comprise an optically detectable label and the optically detectable labels of the first marker (202) and the second marker (204) are optically distinguishable from each other.

13. The method according to any one of the preceding claims, wherein at least a further marker is added to the biological sample (200, 308).

14. The method according to one of the preceding claims, wherein the optical readout is generated by means of a microscope (304).

15. A system (300) configured to perform the method according to one of the preceding claims.
